# EUROPEAN PATENT SPECIFICATION

(11) **EP 3 190 946 B1**
(45) Date of publication and mention of the grant of the patent: **08.05.2019**
(21) Application number: 15766327.9
(22) Date of filing: 04.09.2015
(51) Int. Cl.: A61B 1/05, H01L 27/146, H04N 5/225

(54) **LOW PROFILE CIRCUIT BOARD CONNECTORS FOR IMAGING SYSTEMS**
FLACHE LEITERPLATTENVERBINDER FÜR ABBILDUNGSSYSTEME
CONNECTEURS POUR CARTE DE CIRCUIT MINIATURISÉE POUR SYSTÈMES D'IMAGERIE

(30) Priority: 10.09.2014 US 201462048598 P
(43) Date of publication of application: 19.07.2017
(60) Divisional application: 19166002.6
(73) Proprietor: Cook Medical Technologies LLC, Bloomington, IN 47404 (US)
(72) Inventor: KENNEDY, Kenneth, C., Clemmons, NC 27012 (US)
(74) Representative: Garratt, Peter Douglas
(86) International application number: PCT/US2015/048538
(87) International publication number: WO 2016/040156

(56) References cited:
- JP-A- H0 964 330
- JP-A- S63 240 825
- JP-A- 2006 314 582
- US-A- 4 831 456
- US-A- 5 220 198

## Description

### TECHNICAL FIELD

The present invention relates generally to imaging systems and more particular to a circuit board connector electrically connected to an image sensor, where at least one of opposing planar surfaces of the connector forms an angle with an outer surface of the image sensor that is less than 180 degrees.

### BACKGROUND

Medical procedures may be conducted with the aid of one or more forms of medical imaging. For example, projection images produced by exposing film to x-rays that have passed through a patient may be used for medical diagnostic examinations. Live, or real-time projection x-ray images, known as fluoroscopy, may be used to guide therapeutic medical procedures such as endoscopic retrograde cholangio-pancreatography (ERCP) and balloon angioplasty. Also, Computed Tomography may produce images from x-rays by mathematically reconstructing the x-ray projection data to computationally produce multiple images of a patient in a format of a stack of two-dimensional slices. Ultrasound imaging equipment may produce images for guiding biopsy procedures and for examining both hollow organs and solid tissues of the body.

While x-rays and some other forms of medical imaging rely upon the transmission of electromagnetic radiation or ultrasound waves through a patient, medical endoscopes may provide images from inside a patient by first using visible light to illuminate an internal space within a patient and then using a camera or image sensor to capture that portion of the light that is reflected from the surfaces of the adjacent organs and tissues.

Some cameras are produced as a single integrated circuit (IC), one example being a complementary metal-oxide-semiconductor (CMOS) camera. In general, IC cameras may include an IC that is encased in a larger supporting package that prevents physical damage and corrosion. The package may support a larger set of electrical contacts that are more easily used to connect external cabling and other electrical connections. The larger electrical contacts of the package come in many different forms, one of which is a ball grid array (BGA). A BGA may include solder balls raised above the surface of the package. Inside the protective package, the balls are typically connected to metallized layer contacts of the IC via conductive traces integrated into the package substrate and/or fine wires (wire bonding).

For medical devices that use IC cameras, the electrical contacts of the package connect to conductive cabling that powers the IC camera and communicates signals for operation of the IC camera. A connector may be used to connect the conductors of the cabling with the electrical contacts of the package. To ensure reliable and durable operation of the IC camera, a connector that provides a strong and durable connection between the electrical cabling and the contacts of the IC package may be desirable. Also, since space optimization and size (e.g., outer-diameter) reduction is often desirable for medical device design, a connector that minimizes its axial profile while connecting the electrical cabling with the IC camera may further be desirable.

Reference is directed to US 4,831,456 which discloses an imaging apparatus which includes a camera head and a camera control unit. An objective optical system is arranged at a front end portion of the camera head. A solid-state imaging device having a first external terminal group to or from which signals subjected to signal processing inside the camera head are input or output, and a second external terminal group, which is separated from the first external terminal group, and to or from which signals that are not subjected to signal processing inside the camera head are input or output, is arranged behind the objective optical system. A circuit board has at least two connecting terminal groups. One connecting terminal group of the circuit board is connected to the first external terminal group, and the other connecting terminal group is connected to one end portion of a first conductive wire group of a power cable. One end portion of a second conductive wire group of the power cable is connected to the second external terminal group. The other end portion of each of the first and second conductive wire groups is connected to the camera control unit. Therefore, the camera head can be rendered compact.

Reference is further directed to US 5,220,198 which discloses a solid state imaging apparatus which can be made small in the contour is formed of a solid state imaging device chip provided with pad electrodes formed near a photoelectrically converting part, an internal connecting substrate bent so that a plurality of surfaces not on the same plane as this solid state imaging device chip may be formed and face-bonded and connected with the pad electrodes of this solid state imaging device chip through bumping members and electronic parts fitted on this connecting substrate and functionally connected to the solid state imaging device chip.

Reference is further directed to JP S63 240825, JP H09 64330 and JP 2006 314582.

### BRIEF SUMMARY

In a first aspect, an imaging system may include an image sensor and a circuit board connector. The image sensor may include an outer package having an outer surface, and a plurality of electrical contacts integrated with the outer surface. The circuit board connector may include a first planar surface, a second planar surface opposite the first planar surface, and a plurality of electrical connections. Each of the plurality of electrical connections may include a connection portion that is bonded to one of the plurality of electrical contacts. In addition, each of the connection portions may be disposed on the first planar surface or on the second planar surface. Further, at least one of the first planar surface or the second planar surface may form an angle with the outer surface of the outer package that is greater than zero degrees and less than 180 degrees.

In a second aspect, an endoscopic system may include an elongate tubular member longitudinally extending from a proximal portion to a distal portion, an image sensor, an electrical cabling, and a circuit board connector. The elongate tubular member may include a body, and a cable lumen longitudinally extending in the body from the proximal portion to the distal portion. The image sensor may be disposed in the body at the distal portion and include an outer package having an outer surface facing in a proximal direction, and a plurality of electrical contacts integrated with the outer surface. The electrical cabling may be disposed within the cable lumen and include a plurality of elongate conductive members. The circuit board connector may be disposed in the body at the distal portion and proximal the image sensor. In addition, the circuit board connector may include a first planar surface, a second planar surface opposite the first planar surface, and a plurality of electrical connections electrically connecting the plurality of electrical contacts of the image sensor with the elongate conductive members of the electrical cabling. Each of the plurality of electrical connections may include a connection portion that is bonded to one of the plurality of electrical contacts. In addition, each of the connection portions may be disposed on the first planar surface or on the second planar surface. Further, at least one of the first planar surface or the second planar surface may form an angle with the outer surface of the outer package that is greater than zero degrees and less than 180 degrees.

### BRIEF DESCRIPTION OF THE DRAWINGS

FIG. 1A is an exploded perspective view of an example imaging system.
Fig. 1B is a perspective view of a connector of the example imaging system shown in Fig. 1A from an opposite side.
FIG. 2A is a perspective view of the example imaging system shown in FIG. 1A with the components connected together.
FIG. 2B is a perspective view of the example imaging system shown in FIG. 2A from an opposite side.
FIG. 2C is a side view of an image sensor of the example imaging system shown in FIGS. 1A-2B.
FIG. 2D is an axial view of the image sensor of the example imaging system shown in FIGS. 1A-2B.
FIG. 2E is a top view of the image sensor and a circuit board connector of the example imaging system shown in FIGS. 1A-2B.
FIG. 2F is a side view of the image sensor and the circuit board connector of the example imaging system shown in FIGS. 1A-2B.
FIG. 2G is a perspective view of the image sensor of the example imaging system shown in FIGS. 1A-2B, showing bounds determined by an elliptical axial profile of the image sensor.
FIG. 2H is a perspective view of the image sensor of the example imaging system shown in FIGS. 1A-2B, showing bounds determined by a rectangular axial profile of the image sensor.
FIG. 3A is a perspective view of the example imaging system shown in FIGS. 1A and 2A, further showing bonding material.
FIG. 3B is a perspective view of the example imaging system shown in FIG. 3A from an opposite side.
FIG. 4A is a perspective view of another example imaging system.
FIG. 4B is a perspective view of the example imaging system shown in FIG. 4A from an opposite side.
FIG. 5A is a perspective view of a third example imaging system.
FIG. 5B is a perspective view of the example imaging system shown in FIG. 5A from an opposite side.
FIG. 6A is a perspective view of a fourth example imaging system.
FIG. 6B is a perspective view of the example imaging system shown in FIG. 6A from an opposite side.
FIG. 7 is a partial cross-sectional side view of an example medical system.
FIG. 8A is an exploded side view of an example circuit board and an example image sensor.
FIG. 8B is a side view of the example circuit board and image sensor of FIG. 8A connected together.
FIG. 9A is an exploded side view of another example circuit board and image sensor.
FIG. 9B is a side view of the example circuit board and image sensor of FIG. 9A connected together.
FIG. 9C. is an axial view of the example circuit board and image sensor of FIG. 9A.
FIG. 10 is a top view of another example image sensor implemented with the circuit board connector of the example imaging system shown in FIGS. 1A-2B, where the image sensor has a multi-planar outer surface.
FIG. 11 is a top view of a third example image sensor implemented with the circuit board connector of the example imaging system shown in FIGS. 1A-2B, where the image sensor has a curved outer surface.

### DETAILED DESCRIPTION

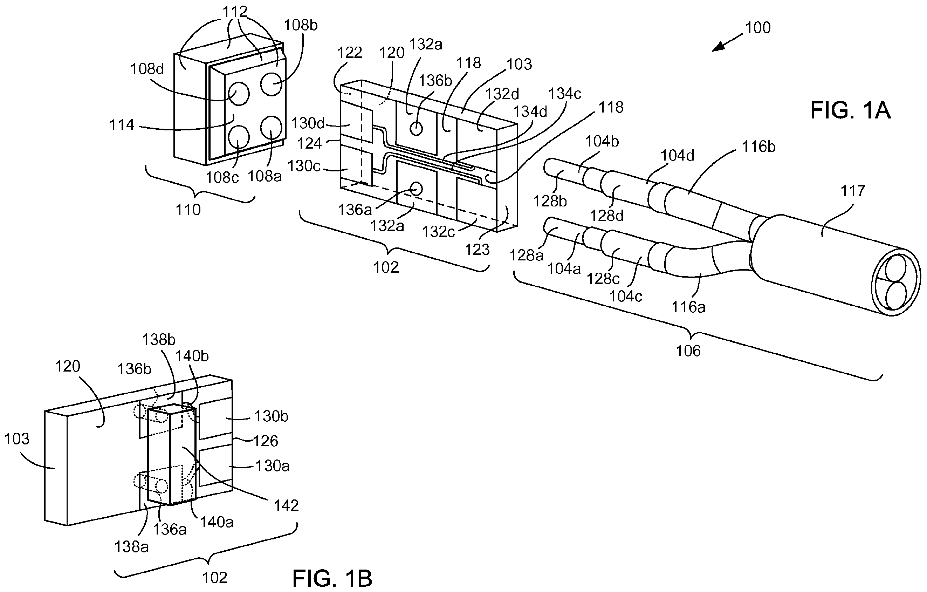

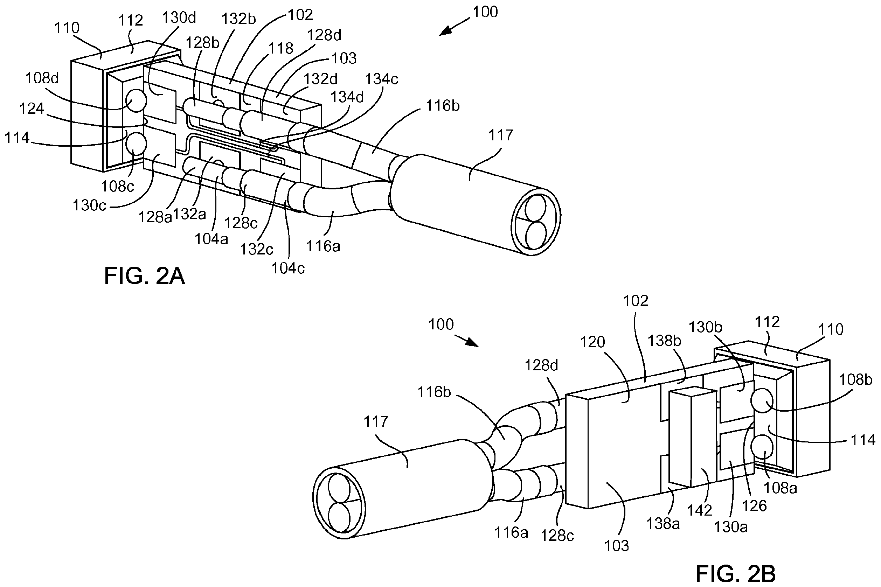

Fig. 1A-2B show an example imaging system 100 that includes a circuit board connector 102 that electrically connects elongate conductive members 104a, 104b, 104c, 104d of an electrical cabling 106 to respective electrical contacts 108a, 108b, 108c, 108d of an image sensor or camera 110. Fig. 1A shows an exploded perspective view of the imaging system 100. Fig. 1B shows a perspective view of the circuit board connector 102 in isolation opposite to the view shown in Fig. 1A. Figs. 2A and 2B show opposite perspective views of the imaging system 100 with the circuit board connector 102, the electrical cabling 106, and the image sensor 110 connected and/or assembled together.

The image sensor 110 may be any known or later developed image sensor that is configured to capture an image of an object. Example image sensors may include those sensors that capture light images from light that is reflected from the object, such as charge coupled-coupled device (CCD) or integrated circuit (IC) sensors such as complementary metal-oxide-semiconductor (CMOS) image sensors, although other types of image sensors may be possible. The image sensor 110 may include inner electronic circuitry, such as one or more chips or integrated circuits (not shown), that is configured to capture images, convert the captured images to electrical signals, and transmit the electrical signals to an external image processor (not shown) for subsequent processing and display of the captured images.

The inner electronic circuitry may be encased and protected by an outer package 112. For purposes of the present description, the outer package 112 may be any structure or combination of structures that encases or houses the inner electronic circuitry. The outer package 112 may be made of any of various materials or combinations of various materials, such as metal, plastic, glass, ceramic as examples. An outer surface 114 of the package 112 may include and/or be integrated with the electrical contacts 108a-108d. For example, the electrical contacts 108a-108d may be disposed or lie on and/or extend or protrude from the outer surface 114.

Each of the electrical contacts 108a-108d may be an input or output for the inner electronic circuitry and communicate signals, such as power, ground, data, and control (e.g., clock) signals, to and from the electronic circuitry for operation of the image sensor 110. In the example configuration shown in Fig. 1, the image sensor 110 has four electrical contacts 108a-108d-a first electrical contact 108a that receives and supplies power to the electronic circuitry, a second electrical contact 108b that is connected to a ground reference for the supplied power, a third electrical contact 108c that outputs the data signals carrying the captured image data received from the inner electronic circuitry, and a fourth electrical contact 108d that receives and supplies a clock signal to the inner electronic circuitry. Alternative example configurations for the image sensor 110 may include more or fewer than four electrical contacts for operation of and communication with the image sensor 110.

The electrical contacts 108a-108d may have a one-dimensional (e.g., linear) or two-dimensional arrangement when integrated with the outer surface 114. One example arrangement may be an M-by-N array or matrix, where M and N are integers. As shown in Fig. 1, the four electrical contacts 108a-108d may be arranged as a two-by-two array. Other one-dimensional or two-dimensional arrangements may be possible.

In addition, the electrical contacts 108a-108d may be of any type of various conductive structures suitable for integration with the outer surface 114 of the outer package 112. Example configurations for the electrical contacts 108a-108d may include solder balls, pins, tabs, or pads as examples. Other conductive structures may be possible. In the example image sensor 110 shown in Fig. 1, the electrical contacts 108a-108d are configured as a two-by-two ball grid array (BGA).

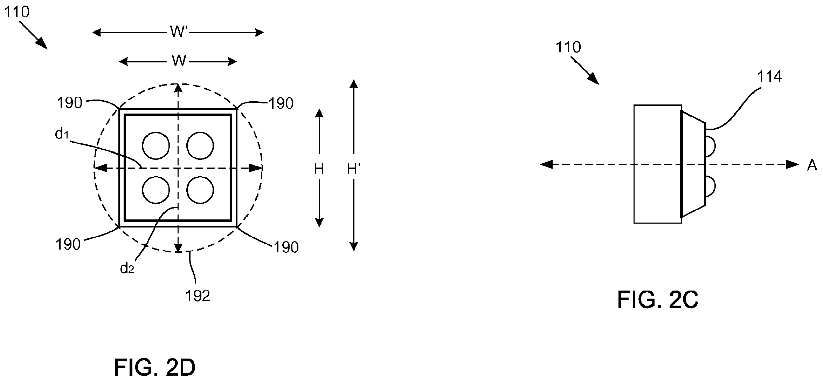

Fig. 2C is a side view of the image sensor 110 shown in isolation from the other components of the example imaging system 100. The image sensor 110 may be oriented relative to an axis A that extends in a direction that is the same as and in a direction that is opposite to the direction in which the outer surface 114 faces.

Fig. 2D is an axial view of the image sensor 110 along the axis A shown in Fig. 2C. The image sensor 110 may have an axial profile with reference to the axis A. The axial profile may define a minimum cross-sectional area that is transverse to the axis A. For some applications, the minimum cross-sectional area may correspond to a minimum cross-section of a space or volume in which the image sensor 110 may be disposed. The axial profile of the image sensor 110 may have different shapes, which may depend on and/or correspond to the application and/or system with which the image sensor 110 is being implemented. One axial profile may be a rectangular axial profile, which may be determined by the width W and the height H of the image sensor 110. Alternatively, the axial profile may be an elliptical or radial axial profile, as identified by the dotted ellipse 192. The size of the elliptical profile may be determined by edges 190 of the image sensor 110. As shown in Fig. 2D, the image sensor 110 may have an effective width W' and an effective height H' associated with the elliptical axial profile. Its effective width W' may correspond to a first diameter d₁ of the dotted ellipse 192, and its effective height H' may correspond to a second diameter d₂ of the dotted ellipse 192. For configurations where the image sensor has a square shape (i.e., its width W and height H are the same), the elliptical axial profile may be circular such that the first and second diameters d₁ and d₂ may be the same.

Referring back to Figs. 1A-2B, the electrical cabling 106 may include four elongate conductive members 104a-104d that are electrically connected to the four electrical contacts 108a-108d. In particular, a first conductive member 104a may be electrically connected to and supply power to the first electrical contact 108a. A second conductive member 104b may be a ground reference connected to the second electrical contact 108b. A third conductive member 104c may be electrically connected to and communicate data signals carrying captured image data from the image sensor 110 to the external image processor. A fourth conductive member 104d may be electrically connected to the fourth electrical contact 108d and supply the image sensor 110 with a clock signal. The electrical cabling 106 may include more or fewer than four elongate conductive member 104a-104d, which may depend on the configuration of the image sensor 110 and/or how many electrical contacts 108 are used for operation of and communication with the image sensor 110.

Additionally, for the example configuration shown in Fig. 1, the electrical cabling 106 may include a pair of coaxial cables 116a, 116b encased in an outer sheath 117, where each of the elongate members 104a-104d is an inner conductor or an outer conductor for one of the coaxial cables 116a, 116b. In particular, for the example configuration shown in Figs. 1A, 1B, the first conductive member 104a supplying the power is the inner conductor of the first coaxial cable 116a, the third conductive member 104c communicating the data signal is the outer conductor of the first coaxial cable 116a, the second conductive member 104b functioning as the ground reference is the inner conductor of the second coaxial cable 116b, and the fourth conductive member 104d communicating the clock signal is the outer conductor of the second coaxial cable 116b. For other example configurations, the elongate members 104a-104d may be configured differently as inner and outer members of coaxial cables than how they are configured in Figs. 1A, 1B. In still other example configurations, the electrical cabling 106 may include electrical transmission lines other than or in addition to coaxial cables. For example, the elongate members 104a-104d may include four separate wires. Non-limiting examples of other example configurations are shown and described in more detail below.

The circuit board connector 102 may include a circuit board 103 that is a generally planar structure that includes a first planar surface 118 and an opposing second planar surface 120. The perspective view of Fig. 1A shows the first planar surface 118. The perspective view of Fig. 1B shows the opposing, second planar surface 120. In general, the circuit board 103 may be any planar structure that includes one or more electrical connections disposed on and/or within a base substrate. Non-limiting examples of the circuit 103 may include printed circuit boards, rigid circuit boards, or flex circuit boards. For some example configurations, the circuit board 103 may be in an unflexed position such that the first and second planar surfaces 118, 120 are not bent or curved.

The circuit board connector 102 may also include a plurality of side surfaces adjacent each of the first and second planar surfaces 118, 120, including a side surface 122 and an opposing side surface 123. The first planar surface 118 may extend to a first edge 124 where the first planar surface 118 meets the side surface 122 (see Fig. 1A), and the second planar surface 120 may extend to a second edge 126 where the second planar surface meets the side surface 122 (see Fig. 1B).

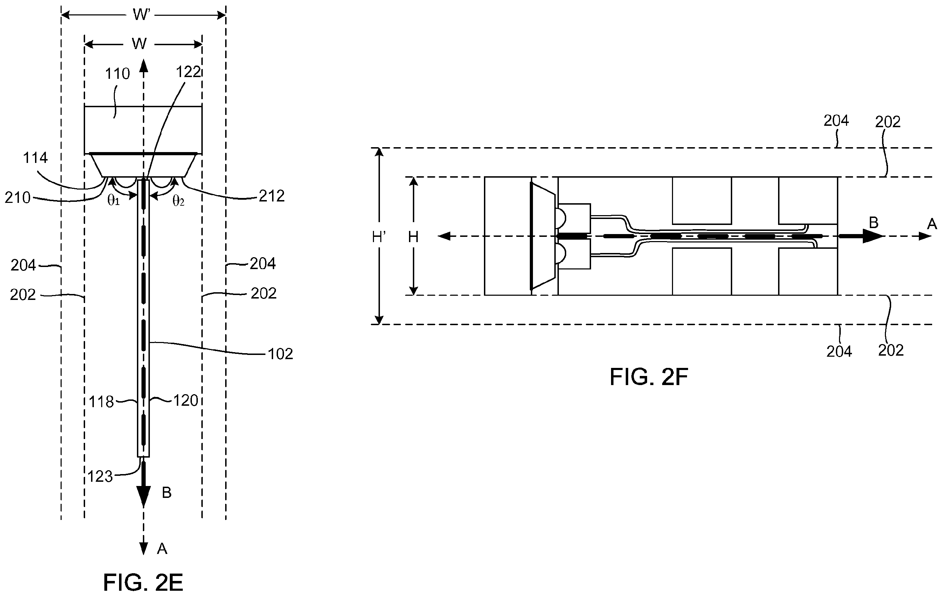

Fig. 2E shows a top view of the image sensor 110 and the circuit board connector 102. Fig. 2F shows a side view of the image sensor 110 and the circuit board connector 102. For some example configurations, as shown in Figs. 2E and 2F, the circuit board connector 102 may extend from the side surface 122 to the opposing side surface 123 in a direction B that is parallel or substantially parallel with the axis A, as denoted by the dotted single arrow B overlaying the dotted double arrow A in Figs. 2E and 2F. Accordingly, the first and second planar surfaces 118, 120 may each face in a direction that is perpendicular or substantially perpendicular to the direction in which the outer surface 114 of the image sensor 110 faces.

In addition, the image sensor 110 and the circuit board connector 102 may have a combined axial profile with reference to the axis A. For some example configurations, the width and thickness of the circuit board connector 102 may be sized such that by orienting the circuit board connector 102 to extend from the first side surface 122 to the second side surface 123 in the same direction that the outer surface 114 faces, the combined axial profile is not bigger than the axial profile of the image sensor 110 alone. As shown in Figs. 2E and 2F, the circuit board connector 102 stays within the bounds 202 of the rectangular axial profile as determined by the width W and height H of the image sensor 110. In addition, the circuit board connector 102 stays within the bounds 204 of the elliptical axial profile as determined by the effective width W' and the effective height H'.

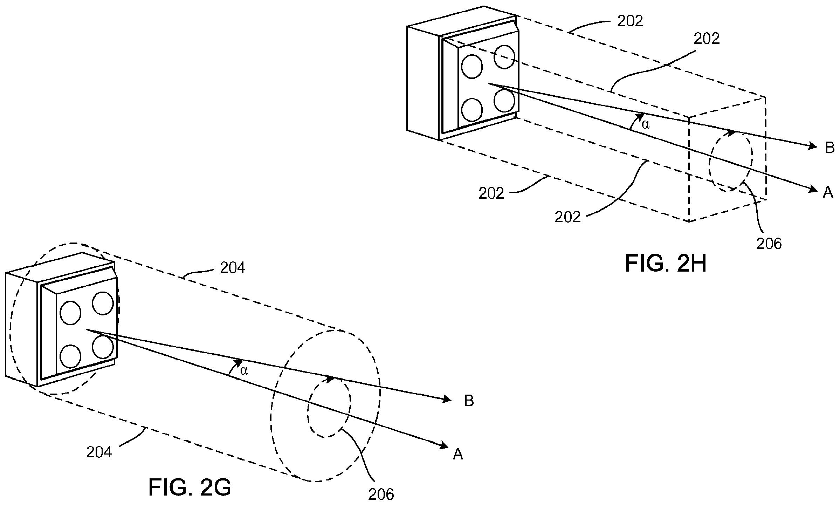

For other example configurations, the circuit board connector 102 may extend from the first surface 122 to the second surface 123 in a direction other in the same direction that the outer surface 114 faces, as is shown in Figs. 1A-2F. Referring to Figs. 2G and 2H, the direction B in which the circuit board extends may be offset by an angle α relative to the axis A. For applications where the combined axial profile is to be minimized, the angular offset α may be chosen such that the circuit board connector 102 stays at least within the bounds 204 of the elliptical axial profile (Fig. 2G), and for some example configurations, also within the bounds 202 of the rectangular axial profile (Fig. 2H). The dotted circular arrow 206 shown in Figs. 2G and 2H denotes that the direction B in which the circuit board connector 102 extends and the axis A may form the angular offset α in any radial direction from the axis A.

Referring back to Fig. 2E, the circuit board connector 102 may be oriented relative to the image sensor 110 such that the direction in which the side surface 122 faces relative to the direction in which the outer surface 114 faces may correspond to the direction B in which the circuit board connector 102 extends. For example, where the direction B is parallel with the direction in which the outer surface 114 faces, then the side surface 122 may face the outer surface 114. That is, the side surface 122 may face in a direction that opposes the direction in which the outer surface 114 faces. Alternatively, if the direction B is offset by the angular offset α as described above with reference to Figs. 2G and 2H, then the direction in which the side surface 122 faces relative to the outer surface 114 may be correspondingly offset.

In addition, the circuit board connector 102 may be oriented relative to the image sensor 110 such that the first planar surface 118 and a first portion 210 of the outer surface 114 form or determine a first angle θ₁, and the second planar surface 120 and a second portion 212 of the outer surface 114 form or determine a second angle θ₂. For example configurations where the direction B in which the circuit board connector 102 extends is parallel with the axis A in which the outer surface 114 faces, the first angle θ₁ and the second angle θ₂ may each be about 90 degrees. However, for other example configurations where the circuit board connector extends at angular offset α relative to the axis A, as previously described with reference to Figs. 2G and 2H, depending on the radial orientation of the angle α relative to the axis A, one of the first angle θ₁ and the second angle θ₂ may be proportionately less than 90 degrees while the other of the first angle θ₁ and the second angle θ₂ may be proportionately greater than 90 degrees. Regardless of whether the first and second angles θ₁, θ₂ are the same or different, the sum of the first and second angles θ₁, θ₂ may be about 180 degrees since the outer surface 114 is a generally flat, planar surface. In other words, the first portion 210 and the second portion 212 are generally oriented in the same plane.

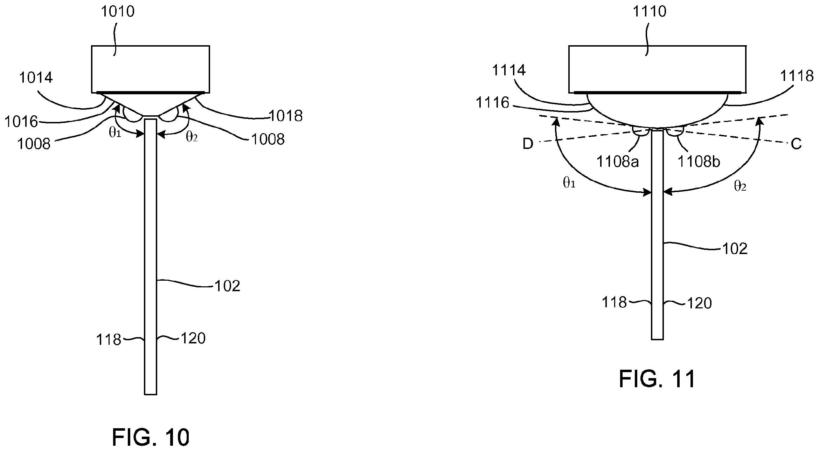

The present description further contemplates the circuit board connector 102 being implemented with image sensors having electrical contacts integrated with an outer surface that is not a generally flat, planar surface. For example, referring to Fig. 10, an example image sensor 1010 may include a plurality of electrical contacts 1008 integrated with an outer surface 1014 that has a first surface portion 1016 oriented in a first plane and a second surface portion 1018 oriented in a second, different plane. The first planar surface 118 of the circuit board connector 102 and the first surface portion 1016 may form or determine a first angle θ₁, and the second planar surface 120 of the circuit board connector 102 and the second surface portion 1018 may form or determine a second angle θ₂. As shown in Fig. 10, the first angle θ₁ and the second angle θ₂ may each be in between 90 degrees and 180 degrees. Otherwise stated, the sum of the first and second angles θ₁, θ₂ may be greater than 180 degrees and less than 360 degrees.

Contours of the outer surface of an image sensor with which electrical contacts are integrated other than planar may be possible. For example, referring to Fig. 11, an image sensor 1110 may have a curved outer surface 1114. The first planar surface 118 may form a first angle θ₁ with a first surface portion 1116 of the outer surface 1114, and the second planar surface 120 may form a second angle θ₂ with a second surface portion 1118. As shown in Fig. 11, the first angle θ₁ may be determined with reference to a plane C that is generally tangential to the portion of the first surface portion 1116 with which an electrical contact 1108a is integrated. Similarly, the second angle θ₂ may be determined with reference to a plane D is generally tangential to the portion of the second surface portion 1118 with which an electrical contact 1108 be is integrated.

In general, at least one of the planar surfaces 118, 120 of the circuit board connector 102 may form or determine an angle θ with an outer surface of an image sensor with which electrical contacts of the image sensor are integrated, where the angle θ is greater than zero and less than 180 degrees.

Referring back to Figs. 1A-2B, the circuit board connector 102 may include a plurality of electrical connections or conductive paths, including a power supply connection, a ground connection, a data signal connection, and a clock signal connection, that electrically connect one of the elongate members 104a-104d with a respective one of the electrical contacts 108a-108d. Each of the electrical connections may include one or more of various conductive structures implemented in a circuit board, such as conductive traces, pads, and vias as examples. Each of the elongate members 104a-104d may have an elongate member end 128a-128d that is electrically connected and bonded to a portion of one of the electrical connections of the circuit board connector 102. Similarly, each of the electrical contacts 108a-108d may be electrically connected and bonded to a portion of one of the electrical connections.

For some example configurations, the portions of the electrical connections that the elongate member ends 128a-128d and the electrical contacts 108a-108d are electrically connected and bonded to may be ends or end portions of the electrical connections. In one example configuration, as shown in Figs. 1A-2B, the electrical contacts 108a-108d may each be electrically connected and bonded to one of first connection ends 130a-130d of the electrical connections, and the elongate member ends 128a-128d may each be electrically connected and bonded to one of second connection ends 132a-132d of the electrical connections. Each electrical connection may extend from one of the first connection ends 130a-130d to a respective one of the second connection ends 132a-132d.

For the example circuit board connector 102, as shown in Figs. 1A-2B, each of the first connection ends 130a-130d and the second connection ends 132a-132d may be conductive contact pads sized appropriately for bonding with a respective one of the elongate member ends 128a-128d or electrical contacts 108a-108d, although other conductive structures may be possible.

Each of the first connection ends 130a-130d may be disposed on the first planar surface 118 or the second planar surface 120. For some example configurations, the first connection ends 130a-130d may be aligned with one of the electrical contacts 108a-108d. Also, the first connection ends 130a-130d disposed on the first planar surface 118 may be positioned at, near, or along the first edge 124, and the first connection ends 130a-130d disposed on the second planar surface 120 may be at, near, or along the second edge 126. As a result, each of the first connection ends 130a-130d may be adjacent or next to one of the electrical contacts 108a-108d of the image sensor 110.

The side surface 122 may be positioned proximate to the outer surface 114 so that the adjacent positioning of the first connection ends 130a-130d and the electrical contacts 108a-108d may be optimized. For some example configurations, the side surface 122 may abut and/or contact the outer surface 114. For other example configurations, a material, such as an adhesive material that affixes the side surface 122 to outer surface 114, may be disposed in between the side surface 122 and the outer surface 114. For still other example configurations, there may be a space or gap in between the side surface 122 and the outer surface 114. Various configurations or combinations of configurations may be possible.

In addition, the arrangement of the first connection ends 130a-130d on the first and second planar surface 118, 120 may depend on the one or two-dimensional arrangement of the electrical contacts 108a-108d and the positioning of the side surface 122 and the edges 124, 126 relative to the electrical contacts 108a-108d. When the electrical contacts 108a-108d are arranged one-dimensionally or linearly, the electrical contacts 108a-108d may be adjacent to only one of the first and second edges 124, 126, and the connection ends 130a-130d may be disposed only on one of the planar surfaces 118, 120. Alternatively, when the electrical contacts 108a-108d are arranged two-dimensionally, at least one of the electrical contacts 108a-108d may be disposed adjacent to the first edge 124 and the other electrical contacts 108a-108d may be disposed adjacent to the second edge 126. When the electrical contacts 108a-108d are disposed adjacent to both of the edges 124, 126, the side surface 122 may be considered to intersect the two-dimensional arrangement of electrical contacts 108a-108d.

In general, the number of first connection ends 130a-130d disposed on the first planar surface 118 may be equal to or correspond to the number of electrical contacts 108a-108d adjacent to the first edge 124. Similarly, the number of first connection ends 130a-130d disposed on the second planar surface 120 may be equal to or correspond to the number of electrical contacts 108a-108d adjacent to the second edge 126.

For the example imaging system 100, the electrical contacts 108a-108d are configured as a two-by-two array, as previously described. As shown in Figs. 2A and 2B, the side surface 122 may intersect the two-by-two array such that the third and fourth electrical contacts 108c, 108d are adjacent to the first edge 124 and the first and second electrical contacts 108a, 108b are adjacent to the second edge 126. Accordingly, first connection ends 130a, 130b may be disposed on the second planar surface 120 and disposed at or near the second edge 126 such that they are adjacent to the first and second electrical contacts 108a, 108b, respectively. Similarly, first ends 130c, 130d may be disposed on the first planar surface 118 and disposed at or near the first edge 124 such that they are adjacent to the third and fourth electrical contacts 108c, 108d, respectively.

The paths that each of the electrical connections take and the conductive structures used to form the paths may depend on whether the first connection ends 130a-130d and the second connection ends 132a-132d are disposed on the first planar surface 118 or the second planar surface 120. For the example connector 102, as shown in Figs. 1A and 2A, the second connection ends 132a-132d are all disposed on the first planar surface 118. To respectively connect the first connection ends 130c, 130d with the second connection ends 132c, 132d, conductive traces 134c, 134d may be disposed on the first planar surface 118. In addition, because the first connection ends 130a, 130b are disposed on the second planar surface 120-i.e., because the first connection ends 130a, 130b and the second connection ends 132a, 132b are disposed on opposing planar surfaces-the electrical connections connecting the first connection ends 130a, 130b with the second connection ends 132a, 132b may also include conductive vias 136a, 136b extending in between the first and second planar surfaces 118, 120. As shown in Figs. 1A and 1B, each of the vias 136a, 136b may extend from a respective second connection end 132a, 132b to a respective pad 138a, 138b or other conductive structure disposed on the second planar surface 120. Conductive traces 140a, 140b disposed on the second planar surface 120 may then extend from a respective one of the pads 138a, 138b to a respective one of the first connection ends 130a, 130b to complete the electrical connections. Various other types of electrical connections may be formed on or in the circuit board connector 102 to electrically connect the first connection ends 130a-130d with the second connection ends 132a-132d.

As shown in Figs. 1B and 2B, the example imaging system 100 may also include a capacitor 142 connected in between the power supply and the ground connections to suppress high frequency and/or noise components. The capacitor 142 may be a surface mount capacitor that is connected and/or bonded to the circuit board connector 102. In particular, the capacitor 142 may have a first end electrically connected and bonded to the contact pad 138a and a second end electrically connected and bonded to the contact pad 138b.

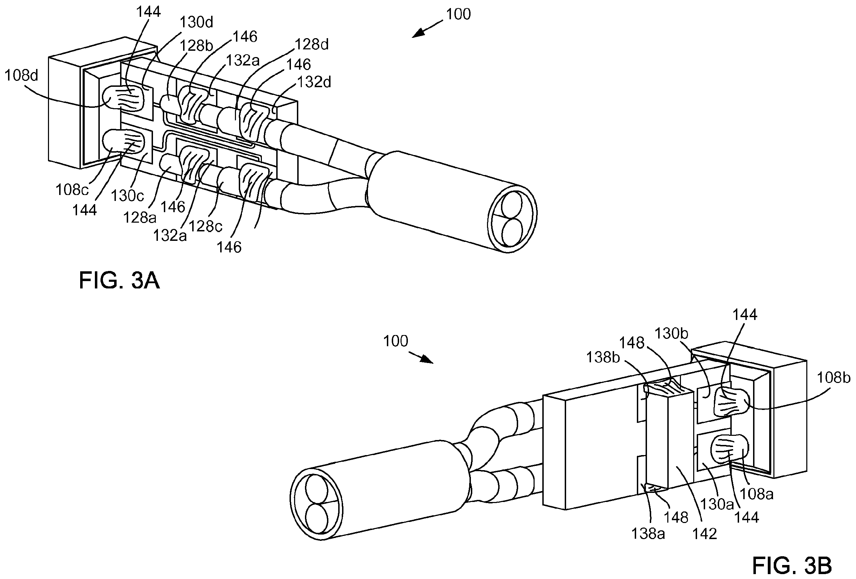

Figs. 3A and 3B are perspective views of the example imaging system 100, further showing additional bonding material 144 that bonds the electrical contacts 108a-108d with respective first connection ends 130a-130d; bonding material 146 that bonds elongate member ends 128a-128d with the second connection ends 132a-132d, and bonding material 148 that bonds the ends of the capacitor 142 to the pads 138a, 138b. Example bonding material 144-148 may include solder, such as reflow solder or conductive adhesive, although other material and/or other bonding techniques such as bond wires may be used to bond and electrically connect the conductive components together. For example, in addition or alternatively to using solder, bond wires may be used to bond and electrically connect the electrical contacts 108a-108d with the first connection ends 130a-130d. Various ways to bond and electrically connect the conductive components together may be possible.

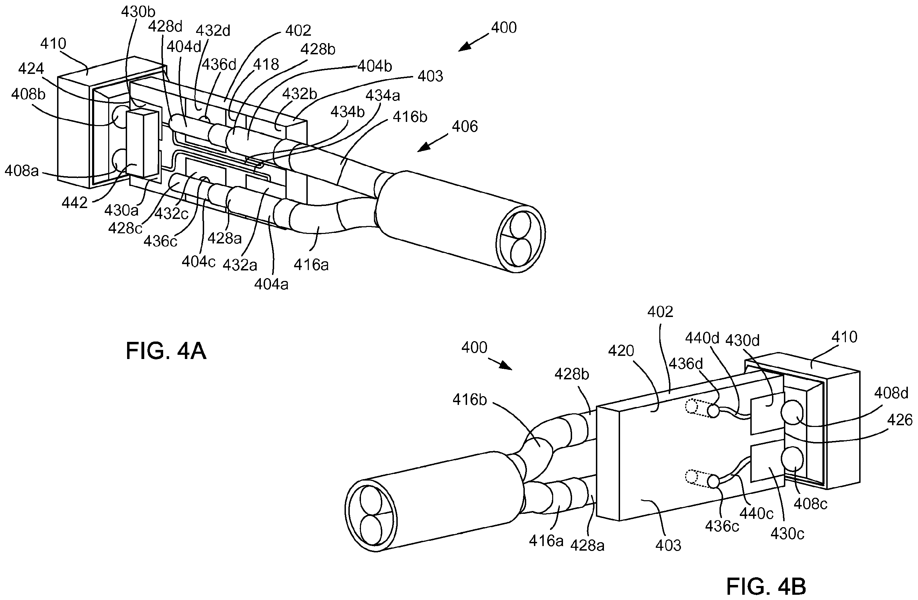

Figs. 4A and 4B show perspective views of another example imaging system 400 that includes a circuit board connector 402 electrically connecting electrical cabling 406 with an image sensor 410. The visualization system 400 is similar to the visualization system 100, except that instead of having the elongate member ends 128a-128d bonded to the first planar surface 118 that extends to the first edge 124 which is adjacent to the data and clock electrical contacts 108c, 108d, the example visualization system 400 has elongate member ends 428a-428d bonded to a circuit board 403 having a first planar surface 418 that extends to a first edge 124 that is adjacent to power and ground electrical contacts 408a, 408b. Additionally, an opposing, second planar surface 420 extends to a second edge 426 that is adjacent to data and clock electrical contacts 408c, 408d.

In the example circuit board connector 402, edge-aligned first ends 430a, 430b may be disposed adjacent and bonded to the power and ground contacts 408a, 408b. Conductive traces 434a, 434b may be disposed on the first planar surface 418 to electrically connect the first connection ends 430a, 430b with second connection ends 432a, 432b, respectively. The elongate member ends 428a, 428b of the conductive elongate members 404a, 404b carrying the power and ground reference signals may be bonded and electrically connected to the second connection ends 432a, 432b, respectively.

A capacitor 442 may be bonded to the same first planar surface 418 to which the elongate member ends 428a-428d are bonded. In the example imaging system 400, the ends of the capacitor 442 are bonded and electrically connected to the edge-aligned first ends 430a, 430b, although configurations involving additional conductive structures such as pads may be possible.

The electrical connections of the circuit board connector 402 that are electrically connected to the elongate members 404c, 404d communicating the data and clock signals may include second connection ends 432c, 432d that are bonded to the elongate member ends 428c, 428d, respectively. Vias 436c, 436d may extend from the second connection ends 432c, 432d to the second planar surface 420. Conductive traces 440c, 440d disposed on the second planar surface 420 may electrically connect the vias 436a, 436b with edge-aligned first connection ends 430c, 430d. Although not shown, other conductive structures, such as contact pads, may also be disposed on the second planar surface 420 as part of the electrical connections.

Similar to the imaging system 100, elongate conductive members 404a-404d may be implemented as a pair of coaxial cables 416a, 416b each having inner and outer conductors. For the example imaging system 400, the elongate conductive members 404a, 404b communicating the power and ground signals may be outer conductors for the coaxial cables 416a, 416b, respectively, and the elongate conductive members 404c, 404d communicating the data and clock signals may be inner conductors for the coaxial cables 416a, 416b, respectively. Other configurations of the cabling 406 may be possible.

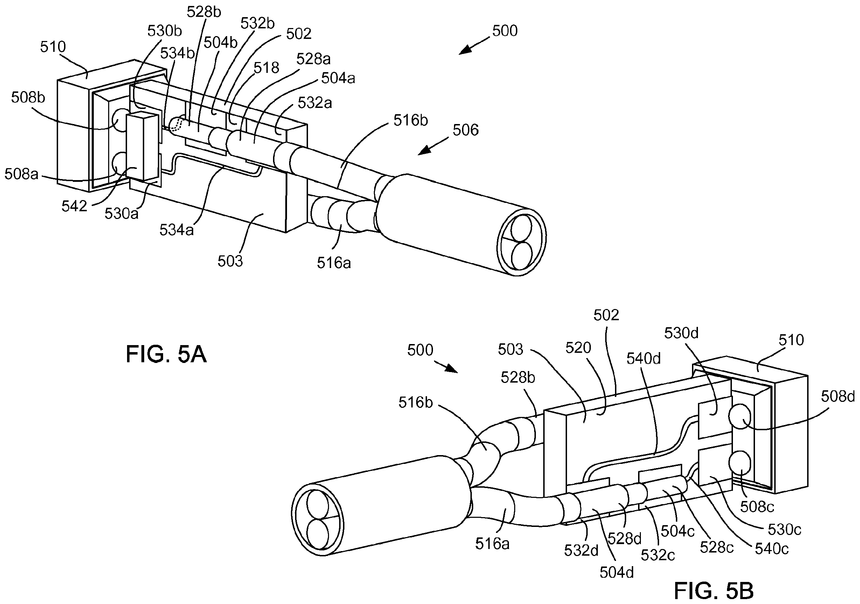

Figs. 5A and 5B show perspective views of another example imaging system 500 that includes a circuit board connector 502 electrically connecting electrical cabling 506 with an image sensor 510. For the example imaging system 500, all of the elongate member ends 528a-528d of the elongate conductive member 504a-504d may not be bonded to the same planar surface of a circuit board 503 of the circuit board connector 502, as is the case for the example imaging systems 100 and 400. That is, some of the elongate member ends 528a-528d may be bonded to a first planar surface 518, while the other elongate member ends 528a-528d may be bonded to an opposing, second planar surface 520.

For the example imaging system 500, elongate member ends 528a, 528b communicating power and ground signals may be bonded to second connection ends 532a, 532b disposed on the first planar surface 518, and elongate member ends 528c, 528d communicating data and clock signals may be bonded to second connection ends 532c, 532d disposed on the second planar surface 520. To complete the electrical connections, conductive traces 534a, 534b disposed on the first planar surface 518 may electrically connect the second connections ends 532a, 532b to respective edge-aligned first connection ends 530a, 530b disposed on the first planar surface 518. The first connection ends 530a, 530b may be bonded to power and ground electrical contacts 508a, 508b. Conductive traces 540c, 540d disposed on the second planar surface 520 may electrically connect the second connection ends 532c, 532d to respective edge-aligned first connection ends 530c, 530d disposed on the second planar surface 520. The first connection ends 530c, 530d may be bonded to data and clock electrical contacts 508c, 508d. Additionally, as shown in Fig. 5A, a capacitor 542 may be connected to the power and ground connections, such as by being bonded to the first connection ends 530a, 530b, although other ways to connect the capacitor 542 to the power and ground connections on the circuit board connector 502 may be possible.

Similar to the example imaging systems 100 and 400, the elongate conductive members 504a-504d may be implemented as coaxial cables 516a, 516b. For the example imaging system 500, the elongate conductive members 504a, 504b communicating power and ground signals may be the outer and inner conductors for the coaxial cable 516b, and the elongate conductive members 504c, 504d may be inner and outer conductors for the coaxial cable 516a. Other ways to implement the elongate conductive members 504a-504d so that they may be bonded to both the first planar surface 518 and the second planar surface 520 may be possible.

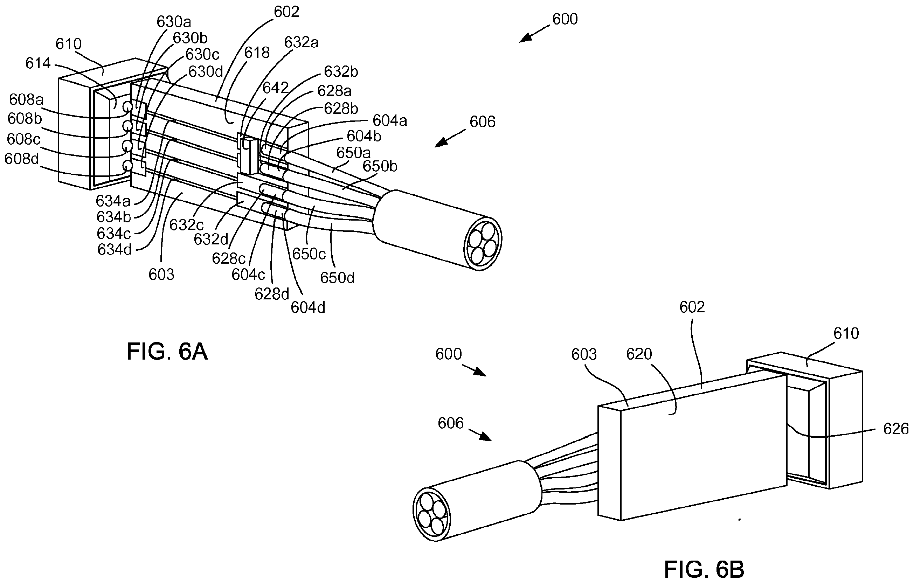

Figs. 6A and 6B show perspective views of another example imaging system 600 in which an image sensor 610 includes power, ground, data, and clock electrical contacts 608a-608d implemented one-dimensionally or linearly as a four-by-one array with an outer surface 614. A circuit board connector 602 may include a circuit board 603 having a first planar surface 618 and an opposing, second planar surface 620. Four edge-aligned first connection ends 630a-630d may be disposed on the first planar surface 618 such that each of the first connection ends 630a-630d are disposed adjacent to one of the electrical contacts 608a-608d. In addition, as shown in Fig. 6B, since none of the electrical contacts 608a-608d are disposed adjacent a second edge 626, none of the first connection ends 630a-630d may be disposed on the second planar surface 620.

Additionally, as shown in Fig. 6A, four second connection ends 632a-632d may be disposed on the first planar surface 618. For other configurations, some or all of the second connection ends 632a-632d may be disposed on the second planar surface 620, with vias extending to the first planar surface 618 to complete the electrical connections. Ends 628a-628d of elongate conductive members 604a-604d may be electrically connected and bonded to the second connection ends 632a-632d. In addition, conductive traces 634a-634d may be disposed on the first planar surface 618 to electrically connect one of the first connection ends 630a-630d to a respective one of the second connection ends 632a-632d so that each of the elongate members 604a-604d may be electrically connected to a corresponding electrical contact 608a-608d.

For the example imaging system 600, the four elongate members 604a-604d are implemented as separate wires, each encased in an insulating coating 650a-650d. Other ways to implement the four connections, such as the coaxial configurations shown and described above, may be possible. Also, as shown in Fig. 6A, a capacitor 642 may be coupled to the power and ground connections, such as by being bonded to the second connection ends 632a, 632b as an example. Other ways may be possible.

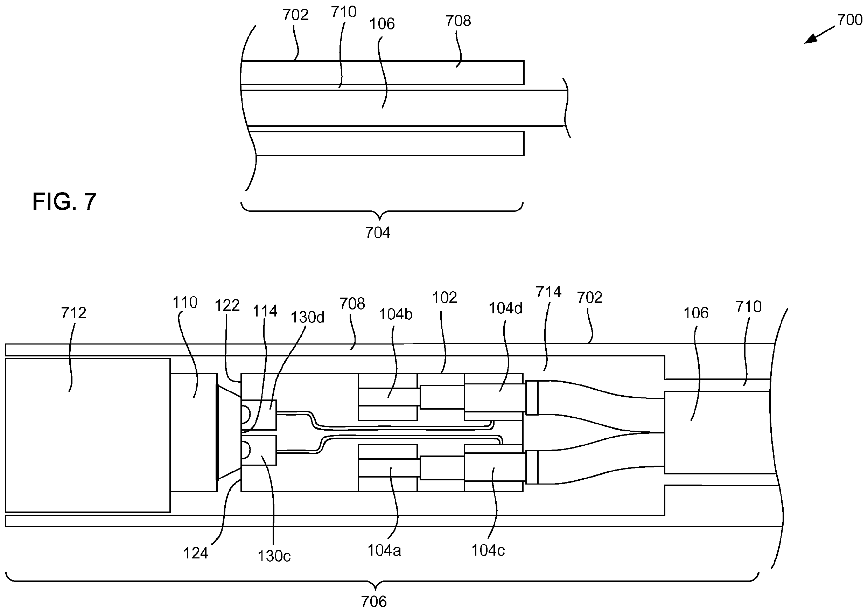

Fig. 7 shows a partial cross-sectional side view of an example medical system 700, such as an example endoscopic medical system, that includes an elongate tubular member 702, such as an endoscope, extending from a proximal portion 704 to a distal portion 706. The example imaging systems 100, 400, 500, 600 previously described may disposed within a body 708 of the elongate tubular member 702 at the distal portion 706. Fig. 7 shows the example imaging system 100, although any of the other example image systems 400, 500, 600 may be similarly implemented with the elongate tubular member 702 as part of the medical system 700.

When implemented in the example medical system 700, various components of the imaging system 100 may be referred to as being oriented distally and proximally. For example, the image sensor 110 may be positioned distal the circuit board connector 102. In addition, the outer surface 114 may face in a proximal direction while the side surface 122 of the circuit board connector 102 may face the outer surface 114 in a distal direction. Further, the first and second edges 124, 126 may be distal edges of the circuit board connector 102, and the first connection ends 130a-130d may be positioned at or near the distal edges 124, 126. Also, as shown in Fig. 7, the imaging system 100 may further include a lens stack 712, which may be disposed distal the image sensor 110 and opposite the outer surface 114. The lens stack 710 may be used to capture and focus light before it reaches the image sensor 110.

Additionally, the elongate tubular member 702 may include a cabling lumen 710 longitudinally extending in the body 708 from the proximal portion 704 to the distal portion 706. The cabling 106 may extend within the cabling lumen 710 from the proximal portion 704 to the distal portion 706. At the distal portion706, the elongate conductive members 104a-104d may be bonded and electrically connected to the circuit board connector 102. Although not shown, the cabling 106 may proximally extend to a proximal end, where the cabling may be electrically coupled to electronic circuitry, such as a buffer circuit, amplification circuitry, a power supply, a clock generator, and/or an image processor as examples, in order to properly communicate the power, ground, data, and clock signals to and from the image sensor 110.

The circuit board connector 102, the image sensor 110, and the lens stack 712 may be disposed at the distal portion 706 in a space or volume 714 that is in communication with the cabling lumen 710. The space 714 may have an axial cross-section having a particular shape, such as rectangular shape or an elliptical shape as examples. For some example configurations, the cross-sectional shape of the space 714 may correspond to the cross-sectional shape of the lens stack 712 and also determine the shape of the axial profile of the image sensor 110 and the circuit board connector 102. As shown in Fig. 7, for some example configurations, the size of the lens stack 712 may be bigger than the axial profile of the image sensor 110. Accordingly, the lens stack 712, rather than the combined axial profile of the image sensor 110 and the circuit board connector 102, may determine the size of the cross-section of the space 714. The circuit board connector 102 may extend in the space 714 proximal the image sensor 110 such that the circuit board connector 102 does not increase the cross-sectional size of the space 714 beyond the size that is required to house the lens stack 712.

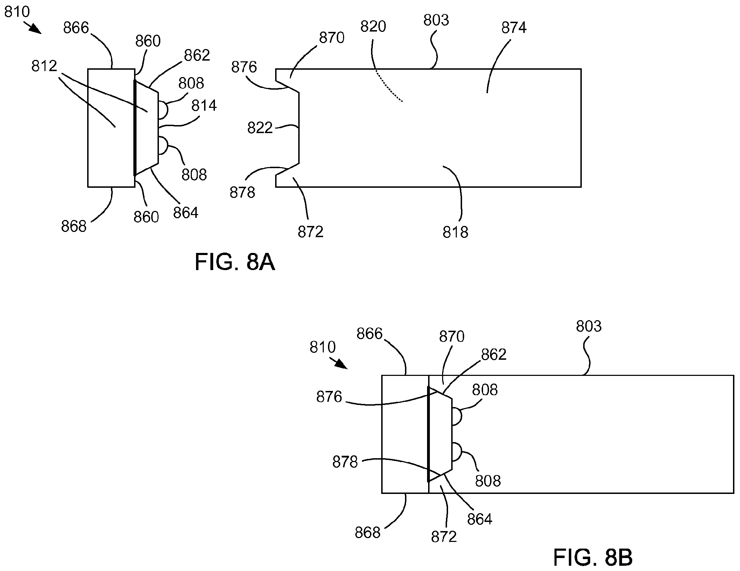

Figs. 8A and 8B show side views of an example circuit board 803 and an image sensor 810. Fig. 8A shows an exploded side view of the circuit board 803 and the image sensor 810. Fig. 8B shows the circuit board 803 and image sensor 810 connected together. The image sensor 810 may be representative of any of the image sensors 110, 410, 510, 610 shown and described with reference to Figs. 1A-7. Additionally, the example circuit board 803 may be used instead of any of the circuit boards 103, 403, 503, 603 for the example circuit board connectors 102, 402, 502, 602 while having configurations of the electrical connections that are similar to those used for the circuit board connectors 102, 402, 502, 602, as previously described.

Referring to Fig. 8A, an outer package 812 may include a first outer surface 814 with which electrical contacts 808 may be integrated. The outer package 812 may also include a base surface 860 oriented generally parallel with the first outer surface 814. The outer package 812 may further include a second outer surface 862 and a third outer surface 864, each extending between the first outer surface 814 and the base surface 860. For the example configuration shown in Fig. 8A, the second and third outer surfaces 862, 864 may each be beveled surfaces relative to the first outer surface 814, although other configurations are possible. For example, the second and third outer surfaces 862, 864 may extend substantially perpendicular to the first outer surface 814, or they may be curved surfaces.

The outer package 812 may further include a fourth outer surface 866 and a fifth outer surface 868 opposing the fourth outer surface 866. The fourth and fifth outer surfaces 866, 868 may each face in directions that are substantially perpendicular to the direction in which the first outer surface 814 faces and also substantially perpendicular to the directions in which a first planar surface 818 and an opposing second planar surface 820 of the circuit board 803 face.

The circuit board 803 may include a pair of alignment wings 870, 872 that each extend from a base portion 874 of the circuit board 803. The alignment wings 870, 872 may extend from the base portion 874 past a side surface 822. Referring to Fig. 8B, each of the alignment wings 870, 872 has a side surface 876, 878 that engages and contacts one of the second and third outer surfaces 862, 864, as shown in Fig. 8B. When the side surfaces 876, 878 of the alignment wings 870, 872 engage the second and third outer surfaces 862, 864, movement of the circuit board 803 relative to the image sensor 810 in a direction perpendicular to directions in which the planar surfaces 818, 820 and the side surface 822 face may be prevented or at least hindered, which may improve stability between the image sensor 810 and the circuit board 803. Further, the alignment wings 870, 872 may be included as part of the circuit board 803 to facilitate aligning first connection ends (not shown in Figs. 8A and 8B) with the electrical contacts 808. That is, when the side surfaces 876, 878 are engaged with the second and third outer surfaces 862, 864, first connection ends may be aligned with and adjacent to the electrical contacts 808.

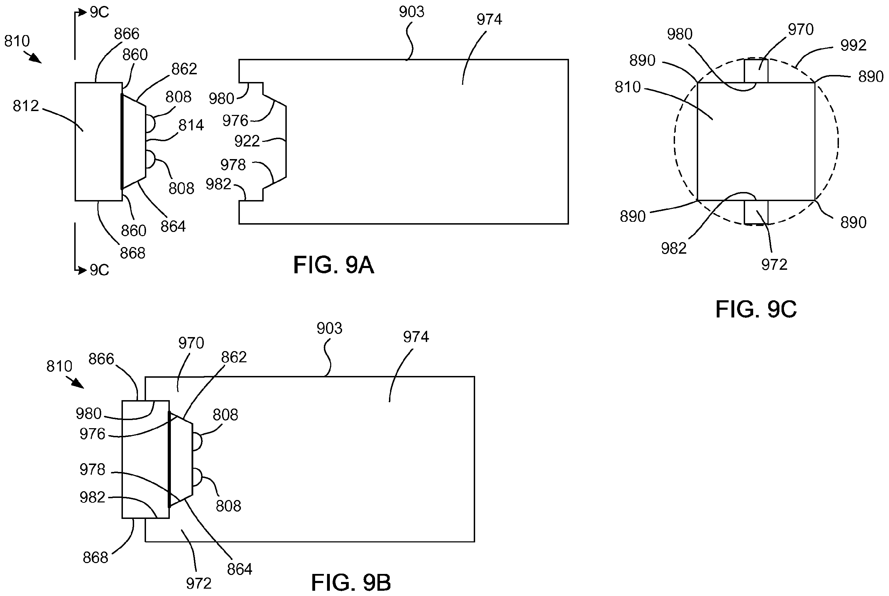

Figs. 9A and 9B show side views of an alternative example circuit board 903 with the image sensor 810. Like the circuit board 803, the example circuit board 903 may be used instead of any of the circuit boards 103, 403, 503, 603 for the example circuit board connectors 102, 402, 502, 602 previously described.

Similar to the circuit board 803, the circuit board 903 may include alignment wings 970, 972 extending from a base portion 974 past a side surface 922. Additionally, like the alignment wings 870, 872, the alignment wings 970,972 may include side surfaces 976, 978 that each engage and contact one of the second and third outer surfaces 862, 864 of the image sensor 810. In contrast to the alignment wings 870, 872, though, the alignment wings 970, 972 may further be configured to have side surfaces 980, 982 that engage and contact the fourth and fifth outer surfaces 866, 868 of the image sensor. By having side surfaces that further engage and contact the fourth and fifth outer surfaces 866, 868, the alignment wings 970, 972 may further enhance stability between the image sensor 810 and the circuit board 920 and alignment capabilities, compared to the alignment wings 870, 872.

Fig. 9C shows an axial view of the image sensor 810 and the circuit board 903 taken along line 9C-9C in Fig. 9A. The dotted circle 992 shows the elliptical axial profile of the image sensor 810, which may be determined by edges 890 of the images sensor 810. Fig. 9C shows that even though the alignment wings 970, 972 expand the width of the circuit board 903 beyond the size of the image sensor 810, the wings 970, 972 may still be within the elliptical axial profile of the image sensor 810. Effectively, then, even with the alignment wings 970, 972, a circuit board connector using the circuit board 903 may not increase the combined elliptical axial profile of the image sensor 810 and circuit board 803, while expanding an overall surface area of the circuit board 903. The increased surface area of the circuit board 903 may be utilized to include more and/or larger circuit components, such as passive circuit elements (resistors, capacitors, etc.). Alternatively to increasing the overall surface area of the circuit board 903, the increased width may enable the length of the circuit board 903 to be shorter without reducing the overall surface area of the circuit board 903, which may reduce the overall rigid length of the imaging system or camera head in which the imaging system is disposed. In addition or alternatively, other configurations of the circuit board 903 may not include the wings 970, 972, such as the circuit boards 103, 403, 503, 603, while still having an increased width that is within the image sensor's elliptical axial profile.

In addition or alternatively to the wings 970, 972, other components of an imaging system or of a related system, such as a medical system to which it is a part of, may be movably or fixedly disposed in the available areas around the image sensor 810 that are within the image sensor's 810 elliptical axial profile, such as cables or light sources as examples.

The foregoing description of various embodiments of the invention has been presented for purposes of illustration and description. It is not intended to be exhaustive or to limit the invention to the precise embodiments disclosed. Numerous modifications or variations are possible in light of the above teachings. The embodiments discussed were chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally, and equitably entitled.

## Claims

1. An imaging system comprising:
an image sensor (110) comprising:
an outer package (112) having an outer surface; and
a plurality of solder balls (108a-108d) configured as a two-dimensional ball grid array integrated with the outer surface; and
a circuit board connector (102) comprising:
a first planar surface (118);
a second planar surface (120) opposite the first planar surface; and
a side surface (122) facing the outer surface that intersects the ball grid array;
a plurality of electrical connections that electrically connect a plurality of elongate conductive members (128a-128d) of electrical cabling (106) with the plurality of solder balls of the ball grid array, the plurality of electrical connections comprising:
a plurality of first connection ends (130a-130d) disposed on the first planar surface and the second planar surface, each first connection end disposed adjacent to, electrically connected to, and bonded to one of the plurality of solder balls; and
a plurality of second connection ends (132a-132d) disposed on at least one of the first planar surface or the second planar surface, each second connection end electrically connected and bonded to a respective one of the plurality of elongate conductive members of the electrical cabling,
wherein at least one of the first planar surface or the second planar surface forms an angle with the outer surface of the outer package that is greater than zero degrees and less than 180 degrees.

2. The imaging system of claim 1, wherein the circuit board connector further comprises:
a first edge where the first planar surface meets the side surface; and
a second edge where the second planar surface meets the side surface,
wherein each of the plurality of solder balls is adjacent to the first edge or the second edge.

3. The imaging system of claims lor 2, wherein a first number of the first connection ends disposed on the first planar surface is equal to a first number of the plurality of solder balls adjacent to the first edge, and wherein a second number of the first connection ends disposed on the second planar surface is equal to a second number of the plurality of solder balls adjacent the second edge.

4. The imaging system of any of claims 1 to 3, wherein at least one of the plurality of electrical connections comprises a conductive via extending from the first planar surface to the second planar surface.

5. The imaging system of any of claims 1 to 4, wherein the outer surface of the outer package comprises a first outer surface, wherein the outer package further comprises at least one second outer surface, and
wherein the circuit board connector further comprises at least one wing that engages with the at least one second outer surface.

6. The imaging system of any of claims 1 to 5, wherein the plurality of electrical connections comprises a power connection configured to communicate a power signal to power the image sensor and a ground connection configured to serve as a ground reference for the power signal, and
wherein the imaging system further comprises a capacitor having a first end bonded to the power connection and a second end bonded to the ground connection.

7. The imaging system of any of claims 1 to 6, wherein the plurality of solder balls comprises at least one of:
a power solder ball configured to communicate a power signal that powers the image sensor;
a ground solder ball configured to communicate a ground reference signal;
a data solder ball configured to communicate a data signal comprising captured image data; or
a clock signal solder ball configured to communicate a clock signal for operation of the image sensor.

8. The imaging system of any of claims 1 to 7, wherein the angle is substantially ninety degrees.

9. The imaging system of any of claims 1 to 7, wherein the angle comprises a first angle, wherein the first planar surface forms the first angle, and wherein the second planar surface forms a second angle with the outer surface, the second angle being greater than zero degrees and less than 180 degrees.

10. The imaging system of any of claims 1 to 9, wherein the image sensor is oriented about an axis that extends in a direction in which the outer surface faces, wherein the image sensor has a first elliptical axial profile, wherein the imaging sensor and circuit board connector combined have a second elliptical axial profile, and wherein the second elliptical axial profile is not greater than the first elliptical axial profile.

## Patentansprüche

1. Abbildungssystem, aufweisend:
einen Bildsensor (110), aufweisend:
eine äußere Verpackung (112) mit einer Außenfläche; und
mehrere Lotkugeln (108a-108d), die als zweidimensionale Kugelgitteranordnung konfiguriert sind, die in die Außenfläche integriert ist; und
einen Leiterplattenverbinder (102), aufweisend:
eine erste planare Fläche (118);
eine zweite planare Fläche (120) gegenüber der ersten planaren Fläche; und
eine der Außenfläche zugewandte Seitenfläche (122), die die Kugelgitteranordnung schneidet;
mehrere elektrischen Verbindungen, die mehrere längliche leitfähige Elemente (128a-128d) der elektrischen Verkabelung (106) mit den mehreren Lotkugeln der Kugelgitteranordnung elektrisch verbinden, wobei die mehreren elektrischen Verbindungen aufweisen:
mehrere erste Verbindungsenden (130a-130d), die auf der ersten planaren Fläche und der zweiten planaren Fläche angeordnet sind, wobei jedes erste Verbindungsende zu einer der mehreren Lotkugeln benachbart angeordnet, mit dieser elektrisch verbunden und gebondet ist; und
mehrere zweite Verbindungsenden (132a-132d), die auf mindestens einer der ersten planaren Fläche oder der zweiten planaren Fläche angeordnet sind, wobei jedes zweite Verbindungsende mit einem jeweiligen der mehreren länglichen leitfähigen Elemente der elektrischen Verkabelung elektrisch verbunden und gebondet ist,
wobei mindestens eine der ersten planaren Fläche oder der zweiten planaren Fläche mit der Außenfläche der äußeren Verpackung einen Winkel bildet, der größer als null Grad und kleiner als 180 Grad ist.

2. Abbildungssystem nach Anspruch 1, wobei der Leiterplattenverbinder ferner aufweist:
eine erste Kante, wo die erste planare Fläche auf die Seitenfläche trifft; und
eine zweite Kante, wo die zweite planare Fläche auf die Seitenfläche trifft,
wobei jede der mehreren Lotkugeln zu der ersten Kante oder der zweiten Kante benachbart ist.

3. Abbildungssystem nach Anspruch 1 oder 2, wobei eine erste Anzahl der ersten Verbindungsenden, die auf der ersten planaren Fläche angeordnet sind, gleich einer ersten Anzahl den mehreren Lotkugeln ist, die zu der ersten Kante benachbart sind, und wobei eine zweite Anzahl der ersten Verbindungsenden, die auf der zweiten planaren Fläche angeordnet sind, gleich einer zweiten Anzahl den mehreren Lotkugeln ist, die zu der zweiten Kante benachbart sind.

4. Abbildungssystem nach einem der Ansprüche 1 bis 3, wobei mindestens eine der mehreren elektrischen Verbindungen eine leitfähige Durchkontaktierung aufweist, die sich von der ersten planaren Fläche zur zweiten planaren Fläche erstreckt.

5. Abbildungssystem nach einem der Ansprüche 1 bis 4, wobei die Außenfläche der Außenverpackung eine erste Außenfläche aufweist, wobei die Außenverpackung ferner mindestens eine zweite Außenfläche aufweist, und
wobei der Leiterplattenverbinder ferner mindestens einen Flügel aufweist, der mit der mindestens einen zweiten Außenfläche in Eingriff steht.

6. Abbildungssystem nach einem der Ansprüche 1 bis 5, wobei die mehreren elektrischen Verbindungen eine Stromverbindung, die konfiguriert ist, ein Stromsignal zum Versorgen des Bildsensors zu übermitteln, und eine Masseverbindung, die konfiguriert ist, als Bezugsbasis für das Stromsignal zu dienen, aufweisen, und
wobei das Abbildungssystem ferner einen Kondensator aufweist, der ein erstes Ende, das an die Stromverbindung gebondet ist, und ein zweites Ende, das an die Masseverbindung gebondet ist, hat.

7. Abbildungssystem nach einem der Ansprüche 1 bis 6, wobei die mehreren Lotkugeln mindestens eines aufweisen von:
eine Strom-Lotkugel, die konfiguriert ist, ein Stromsignal zu übermitteln, das den Bildsensor treibt;
eine Masse-Lotkugel, die konfiguriert ist, ein Massebezugssignal zu übermitteln;
eine Daten-Lotkugel, die konfiguriert ist, ein Datensignal zum übermitteln, das erfasste Bilddaten aufweist; oder
eine Taktsignal-Lotkugel, die konfiguriert ist, ein Taktsignal für den Betrieb des Bildsensors zu übermitteln.

8. Abbildungssystem nach einem der Ansprüche 1 bis 7, wobei der Winkel im Wesentlichen neunzig Grad beträgt.

9. Abbildungssystem nach einem der Ansprüche 1 bis 7, wobei der Winkel einen ersten Winkel aufweist, wobei die erste planare Fläche den ersten Winkel bildet, und wobei die zweite planare Fläche mit der Außenfläche einen zweiten Winkel bildet, wobei der zweite Winkel größer als null Grad und kleiner als 180 Grad ist.

10. Abbildungssystem nach einem der Ansprüche 1 bis 9, wobei der Bildsensor um eine Achse ausgerichtet ist, die sich in einer Richtung erstreckt, in die die äußere Fläche weist, wobei der Bildsensor ein erstes elliptisches axiales Profil hat, wobei der Bildsensor und der Leiterplattenverbinder kombiniert ein zweites elliptisches axiales Profil haben, und wobei das zweite elliptische axiale Profil nicht größer als das erste elliptische axiale Profil ist.

## Revendications

1. Système d'imagerie comprenant :
un capteur d'image (110) comprenant :
un emballage externe (112) possédant une surface externe ; et
une pluralité de billes de soudure (108a-108d) conçues comme une matrice de billes bidimensionnelle intégrée à la surface externe ; et
un connecteur de carte de circuit (102) comprenant :
une première surface plane (118) ;
une seconde surface plane (120) opposée à la première surface plane ; et
une surface latérale (122) faisant face à la surface externe qui croise la matrice de billes ;
une pluralité de connexions électriques qui connectent électriquement une pluralité d'éléments conducteurs allongés (128a-128d) d'un câblage électrique (106) avec la pluralité de billes de soudure de la matrice de billes, la pluralité de connexions électriques comprenant :
une pluralité de premières extrémités de connexion (130a-130d) disposées sur la première surface plane et la seconde surface plane, chaque première extrémité de connexion étant disposée adjacente, connectée électriquement et collée à une de la pluralité de billes de soudure ; et
une pluralité de secondes extrémités de connexion (132a-132d) disposées sur l'une au moins de la première surface plane ou la seconde surface plane, chaque seconde extrémité de connexion étant connectée électriquement et collée à l'un respectif de la pluralité d'éléments conducteurs allongés du câblage électrique ;
dans lequel l'une au moins de la première surface plane ou de la seconde surface plane forme un angle avec la surface externe de l'emballage externe qui est supérieur à zéro degrés et inférieur à 180 degrés.

2. Système d'imagerie selon la revendication 1, dans lequel le connecteur de carte de circuit comprend en outre :
un premier bord où la première surface plane rejoint la surface latérale ; et
un second bord où la seconde surface plane rejoint la surface latérale ;
dans lequel chacune de la pluralité de billes de soudure est adjacente au premier bord ou au second bord.

3. Système d'imagerie selon les revendications 1 ou 2, dans lequel un premier nombre des premières extrémités de connexion disposées sur la première surface plane est égal à un premier nombre de la pluralité de billes de soudure adjacentes au premier bord, et dans lequel un second nombre des premières extrémités de connexion disposées sur la seconde surface plane est égal à un second nombre de la pluralité de billes de soudure adjacentes au second bord.

4. Système d'imagerie selon l'une quelconque des revendications 1 à 3, dans lequel l'une au moins de la pluralité de connexions électriques comprend un trou d'interconnexion conducteur s'étendant de la première surface plane à la seconde surface plane.

5. Système d'imagerie selon l'une quelconque des revendications 1 à 4, dans lequel la surface externe de l'emballage externe comprend une première surface externe, dans lequel l'emballage externe comprend en outre au moins une seconde surface externe, et
dans lequel le connecteur de carte de circuit comprend en outre une ailette qui entre en contact avec l'au moins une seconde surface externe.

6. Système d'imagerie selon l'une quelconque des revendications 1 à 5, dans lequel la pluralité de connexions électriques comprend une connexion de puissance conçue pour communiquer un signal de puissance pour alimenter le capteur d'image et une connexion de mise à la masse conçue pour servir de référence de masse pour le signal de puissance, et
dans lequel le système d'imagerie comprend en outre un condensateur ayant une première extrémité reliée à la connexion de puissance et une seconde extrémité reliée à la connexion de mise à la masse.

7. Système d'imagerie selon l'une quelconque des revendications 1 à 6, dans lequel la pluralité de billes de soudure comprend l'une au moins de :
une bille de soudure de puissance conçue pour communiquer un signal de puissance qui alimente le capteur d'image ;
une bille de soudure de mise à la masse conçue pour communiquer un signal de référence de masse ;
une bille de soudure de données configurée pour communiquer un signal données comprenant des données d'image capturée ; ou
une bille de soudure de signal d'horloge conçue pour communiquer un signal d'horloge pour le fonctionnement du capteur d'image.

8. Système d'imagerie selon l'une quelconque des revendications 1 à 7, dans lequel l'angle est essentiellement de 90 degrés.

9. Système d'imagerie selon l'une quelconque des revendications 1 à 7, dans lequel l'angle comprend un premier angle, dans lequel la première surface plane forme le premier angle, et dans lequel la seconde surface plane forme un second angle avec la surface externe, le second angle étant supérieur à zéro degrés et inférieur à 180 degrés.

10. Système d'imagerie selon l'une quelconque des revendications 1 à 9, dans lequel le capteur d'image est orienté autour d'un axe qui s'étend dans une direction vers laquelle la surface externe fait face, dans lequel le capteur d'image possède un premier profil axial elliptique, dans lequel le capteur d'image et le connecteur de carte de circuit combinés possèdent un second profil axial elliptique, et dans lequel le second profil axial elliptique n'est pas plus important que le premier profil axial elliptique.
